# EUROPEAN PATENT APPLICATION

(11) **EP 4 534 938 A1**
(43) Date of publication of application: **09.04.2025**
(21) Application number: 24181667.7
(22) Date of filing: 12.06.2024
(51) Int. Cl.: F26B 21/00, F26B 25/08

(54) **CABINET AND METHOD FOR DRYING SURGICAL INSTRUMENTS**

(30) Priority: 04.10.2023 US 202363542325 P
(71) Applicant: OLYMPUS WINTER & IBE GMBH, 22045 Hamburg (DE)
(72) Inventor: AEHLIG, Dennis, 22081 Hamburg (DE)
(74) Representative: Seemann & Partner Patentanwälte mbB

(57) **Abstract**

The invention relates to a cabinet 2 and a method for drying surgical instruments 1. The cabinet 2 comprises an interior space 20 with at least one mounting device 23. The mounting device 23 is configured to hold a surgical instrument 1 in a way that a control section 12 of the surgical instrument 1 is arranged at a specified holding position and in a specified orientation. The interior space 20 comprises at least one nozzle 24 connected to a gas source 27 by a supply line 28. The nozzle 24 emits a drying gas stream 26 directly towards the specified holding position, in such a direction that the drying gas stream 26 runs along and in between control knobs 13 of the control section 12.

## Description

The invention relates to Cabinet for drying surgical instruments, in particular endoscopes. The invention further relates to a method for drying surgical instruments.

After they have been used, surgical instruments like endoscopes have to be cleaned and disinfected before they can be used again. After cleaning and disinfecting, the surgical instruments have to be dried before they are stored for further use. This drying can take place, for example, inside a dedicated cabinet for drying and storing the surgical instruments or inside a cabinet of a washing and disinfecting apparatus. In either way, the drying process should remove the water and other liquids from the inner channels as well as from the outer surfaces of the surgical instrument.

Often, the drying of the outer surface is performed by an airflow that in blown over the outer surface in order to speed up the normal drying procedure. However, in order to be used again, the surgical instrument has to be completely dried. This requires that all of the accumulated liquid, for example drops and moisture, is removed from all parts of the outer surface of the surgical instrument. However, there are some parts of the outer surface that take longer to dry than other parts. For example, surgical instruments usually comprise a control section with a number of different shaped control knobs, which are used to control the surgical instrument during an operation. During the cleaning step, the cleaning liquid will accumulate between these control knobs. A laminar air flow will not remove this liquid from the outer surface, as it is difficult to reach. Thus, the total time required to dry the surgical instrument is determined by the time that is required to remove all of the liquid on and in between the control knobs. This can take around two hours.

It is the object the present invention to reduce the drying time of surgical instruments after they have been cleaned and disinfected.

This object is solved by a cabinet for drying surgical instruments, in particular endoscopes, wherein the cabinet comprises an interior space with at least one mounting device, wherein the mounting device is configured to hold a surgical instrument in a way, that a control section of the surgical instrument is arranged at a specified holding position and in a specified orientation, wherein the interior space comprises at least one nozzle, wherein the nozzle is connected to a gas source by a supply line, wherein the gas source is designed to supply a drying gas stream to the nozzle via the supply line, wherein the nozzle is designed and arranged to guide the drying gas stream directly towards the specified holding position, in such a direction that the drying gas stream runs along and in between control knobs of the control section, when the control section is arranged at the specified holding position and in the specified orientation.

By arranging the nozzle in such a way, that it guides the drying gas stream towards the specified holding position, the control knobs of the surgical instrument held in the mounting device are specifically targeted by the drying gas stream. This achieves a much shorter drying time, as the moisture on and in between the control knobs is removed quickly. In this way, the drying time can be reduced to about 30 minutes up from about two hours.

The control section is the part of the surgical instrument used to control the surgical instrument during an operation. The control section comprises the control knobs, which can take the shape of levers, buttons, control wheels or similar control elements. As these control knobs are primarily designed and formed in order to facilitate handling of the surgical instrument during an operation, they often have gaps and other small spaces in between them in which the moisture accumulates after cleaning and disinfecting. The surgical instrument may be an endoscope, in particular a flexible endoscope. The control section of a flexible endoscope may comprise control knobs used to guide the flexible tube of the endoscope during an operation, for example in the form of control wheels or control cogs.

The cabinet may be a dedicated drying cabinet. The cabinet may also be a storage cabinet configured to both dry and store the surgical instrument. The cabinet may also be a chamber of a cleaning and disinfecting apparatus.

The mounting device may be a hanger. The hanger may be used to suspend the surgical instrument inside the interior space in a vertical position. The mounting device may also be formed to accommodate the surgical device in a horizontal position. The mounting device may be formed to at least partially enclose an outer surface of the control section. For example, the mounting device may comprise a holding space arranged in between two sidewalls in which the control section of the surgical instrument may be placed. The mounting device may hold the surgical instrument in a fixed position and a fixed orientation, so that the control section of a specified type of surgical instruments will always be at the same specified holding position and the same specified orientation. In this way, the drying gas stream from the nozzle will dry the moisture in between the control knobs reliably. The mounting device may comprise one or more fixtures to hold the surgical device in the fixed position and the fixed orientation.

Preferably, the cabinet comprises a control element arranged along the supply line, wherein the control element is designed to close and open the supply line, wherein in particular the control element is a valve. With the control element, the drying gas stream from the nozzle can be switched on and off. In this way, the cabinet may be configured to provide the drying gas stream only when necessary, for example when a new surgical instrument is placed in the cabinet that has not been dried yet.

Preferably, the cabinet comprises a control system, wherein the control system is configured to close and open the control element periodically in order to create a pulsed gas stream from the nozzle. The control system may be a computer, a microcontroller or a similar control unit. The pulsed gas stream is advantageous in that is effectively removes the moisture from in between the control knobs. The pulsed gas stream may comprise a single pulse or multiple pulses. The pulsed gas stream may be changed to a continuous gas stream after a specified number of pulses. In this way, the drying process may be finalized after the moisture in between the control knobs has been removed. In particular, a pressure of the drying gas stream higher for the pulsed gas stream in comparison to the continuous gas stream.

The pressure of the pulsed gas stream may for example be 1 bar to 2 bar. The pressure of the continuous gas stream may be higher than a storage pressure. The storage pressure may be higher than an ambient pressure. By using different gas pressures, the drying speed is improved and a recontamination after the drying is prevented.

Preferably, the gas source is an air source designed to supply heated air or unheated air to the nozzle. Air is a readily availably gas which is effective for removing the moisture from in between the gaps of the control knobs. By utilizing heated air, the drying time is further reduced. The air may also be dry air. Dry air is air with a low dew point, for example medical grade air.

Preferably, the gas source supplies ambient air and/or medical grade air and/or a gas composition to the nozzle. Ambient air is readily available and provides an efficient way to dry the outer surface of the surgical instrument. Medical grade air has been purified in order to remove contaminations. Thus, medical grade air is advantageous in that it prevents recontamination of the surgical instrument compared to ambient air. The gas composition may comprise one or more noble gases and/or air. Such noble gases further prevent any recontamination during drying and storage. Preferably, the drying gas stream is a pressurized gas stream. In particular, the pressure of the pressurized gas stream is at least one bar above the ambient air pressure. For example, the pressure of the pressurized gas stream is about two bar above the ambient air pressure. In particular, the pressure of the pressurized gas stream is higher during peaks of the pulsed gas stream than during the continuous gas stream. Higher pressures are more effective in removing any moisture from in between the control knobs.

Preferably, the mounting device is configured to hold the control section at an inclined orientation, such that a longitudinal direction of the surgical instrument at the specified holding position has an angle of 0° to 20° to a vertical direction of the cabinet for a vertical drying cabinet and/or 20° to 45° to the vertical direction of the cabinet for a horizontal drying cabinet. By holding the control section at an inclined orientation, the drying gas stream can remove the moisture in between the control knobs more efficiently. The inclined orientation may be the specified orientation. In particular, a vertical drying cabinet is a cabinet in which the surgical instrument is held in an essentially vertical orientation, while in a horizontal cabinet the surgical instrument is held in an essentially horizontal orientation.

Preferably, the at least one nozzle is arranged at a predetermined distance to the specified holding position, wherein the predetermined distance is smaller than 10 cm, wherein in particular the predetermined distance is 4 cm to 8 cm. By arranging the nozzles this close to the control section, which is arranged at the specified holding position, the drying process is further improved. The predetermined distance to the at least one nozzle is chosen to improve drying efficiency.

Preferably, the at least one nozzle is arranged on a ceiling wall of the interior space. The arrangement of the at least one nozzle on the ceiling wall facilitates the drying process, as the nozzle can be arranged very close to the specified holding position. In other embodiments, the at least one nozzle may be arranged on side walls of the interior space, in particular in an upper quarter of the side walls.

Preferably, the cabinet comprises a temperature control unit configured to control the temperature of the drying gas stream, wherein in particular the temperature control unit is configured to regulate the temperature of the drying gas stream to the dew point of the drying gas stream. By regulating the temperature of the drying gas stream, the drying process can be further improved. The temperature may be regulated to the dew point of the drying gas stream in order to speed up the drying process further. In particular, the cabinet comprises at least one sensor configured to detect a water content of the air and/or gas inside the interior space and/or of the drying gas stream. By using the sensor data of the sensor, the control system may regulate the temperature to the dew point.

In particular, the cabinet comprises an air removal unit configured to remove air and/or gas from the interior space of the cabinet. The air removal unit may be configured to remove the air and/or gas inside the interior space in such a quantity that a specified storage pressure and/or drying pressure is achieved inside the interior space. The air removal unit may be configured to remove access moisture from the air inside the interior space. This allows the cabinet to regulate the moisture of the air inside the interior space during drying and/or storage.

The object is further solved by a method for drying surgical instruments, in particular endoscopes, inside a cabinet, wherein the cabinet comprises an interior space with at least one mounting device, wherein the surgical instrument is arranged in the at least one mounting device in a way, that a control section of the surgical instrument is arranged at a specified holding position and in a specified orientation, wherein a gas source supplies a drying gas stream via a supply line to a nozzle of the interior space, wherein the nozzle guides the drying gas stream directly towards the specified holding position, in such a direction that the drying gas stream runs along and in between control knobs of the control section. The same or similar advantages apply to the method for drying surgical instruments as previously mentioned with respect to the cabinet for drying surgical instruments.

In particular, the surgical instrument is suspended in the at least one mounting device.

The method for drying surgical instruments embodies the same or similar advantages as the previously mentioned cabinet.

According to an embodiment, the method for drying surgical instruments comprises multiple phases. In particular, the method for drying surgical instruments comprises a pulsed phase and/or a continuous phase or a storage phase. During the pulsed phase, the drying gas stream may comprise one or more drying gas pulses emitted from the nozzle. During the continuous phase, the drying gas stream may have a constant pressure. The pulsed phase and the continuous phase may constitute or may be part of a drying phase. During the storage phase, the pressure inside the interior space may be higher than an ambient air pressure and lower than a pressure during the drying phase.

Preferably, the supply line is closed and opened via a control element, in particular a valve, to stop and start the drying gas stream.

Preferably, the control element is closed and opened periodically to create a pulsed gas stream from the nozzle.

Preferably, the gas source is an air source, which supplies heated air or unheated air to the nozzle.

Preferably, the gas source supplies ambient air and/or medical grade air and/or a gas composition to the nozzle.

Preferably, the drying gas stream is a pressurized gas stream.

Preferably, the mounting device holds the control section at an inclined orientation, such that a longitudinal direction of the surgical instrument at the specified holding position has an angle of 0° to 20° to a vertical direction of the cabinet for a vertical drying cabinet and/or 20° to 45° to the vertical direction of the cabinet for a horizontal drying cabinet.

Preferably, the at least one nozzle is arranged at a predetermined distance to the specified holding position, wherein the predetermined distance is smaller than 10 cm, wherein in particular the predetermined distance is 4 cm to 8 cm.

Preferably, the at least one nozzle is arranged on a ceiling wall of the interior space.

Preferably, a temperature control unit controls the temperature of the drying gas stream, wherein in particular the temperature control unit regulate the temperature of the drying gas stream to the dew point of the drying gas stream.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic simplified representation of a control section of a surgical instrument in form of a flexible endoscope,
- Fig. 2: a schematic simplified perspective representation of a cabinet for drying and storing surgical instruments and
- Fig. 3: a schematic representation of a cabinet for drying surgical instruments integrated in an apparatus for cleaning and disinfecting surgical instruments.

In the drawings, the same or similar types of elements or respectively corresponding parts are provided with the same reference numbers in order to prevent the item from needing to be reintroduced.

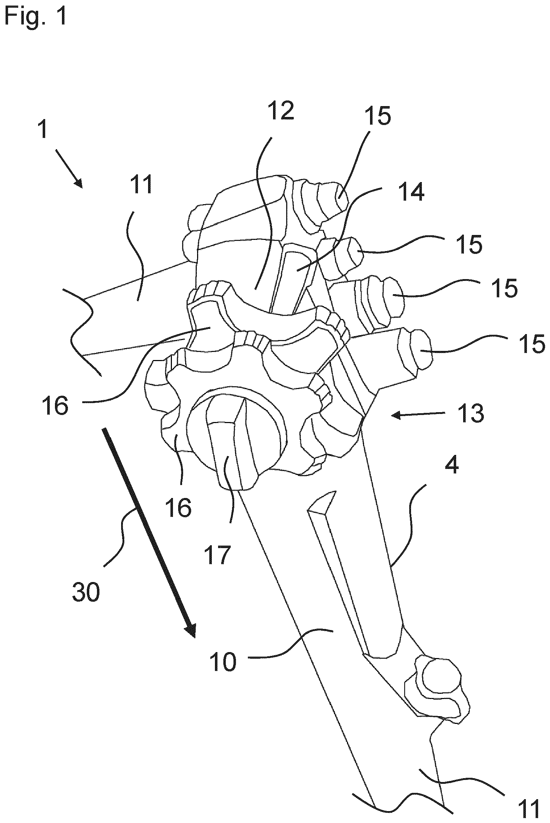

Fig. 1 shows a schematic simplified perspective drawing of a surgical instrument 1 in form of a flexible endoscope 4. The endoscope 4 comprises a handle 10 and a control section 12 as well as two tubes 11, which emerge from the handle 10 and the control section 12. For example, one tube 11 is connected to a light source of the endoscope 4, while the other tube 11 is used for inspecting interior parts of a body during an operation. Along a longitudinal direction 30 of the surgical instrument 1, the control section 12 comprises a number of control knobs 13. In this exemplary embodiment the control knobs 13 comprise a lever 14, several buttons 15, two control cogs 16 and a control wheel 17. These control knobs 13 are used to control the different functions of the endoscope 4 during the operation.

After an operation, the endoscope 4 has to be cleaned and disinfected before it can be used again. Apart from the cleaning of its inner channels, the outer surface of the endoscope 4 also has to be cleaned. Afterwards, the endoscope 4 needs to be dried to remove any remaining moisture, before it can be used again for an operation. However, the time required to completely dry the endoscope 4 is greatly increased by moisture and droplets, which accumulate between the control knobs 13. This remaining liquid takes much longer to dry than the rest of the outer surface and is not removed by laminar air flows often used in cabinets.

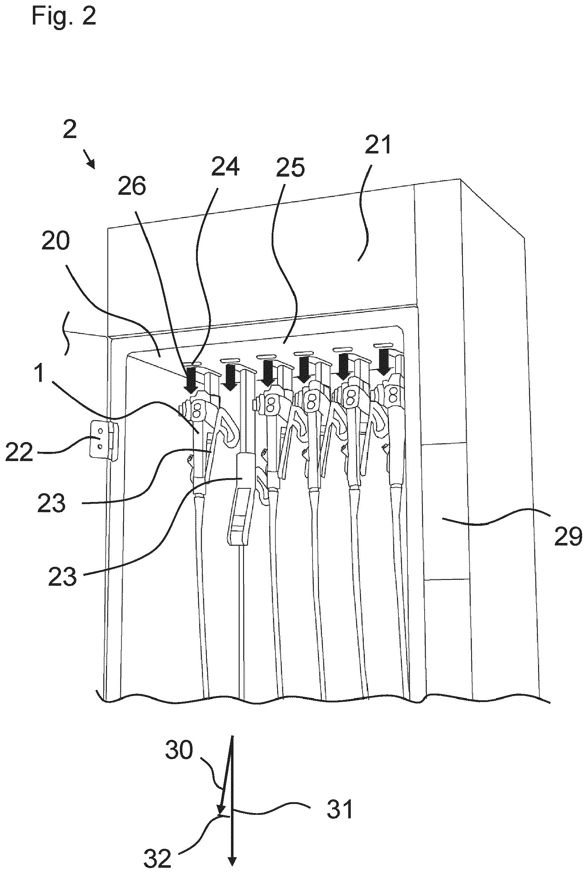

Fig. 2 shows an exemplary embodiment of a cabinet 2 for drying and storing surgical instruments 1 after cleaning and disinfecting them. The cabinet 2 in this embodiment is a vertical drying cabinet. It comprises a housing 21 and a door 22, which enclose an interior space 20. Inside the interior space 20, there are a number of mounting devices 23 configured to hold the handles 10 and/or control sections 12 of the surgical instruments 1. In the shown embodiment, the mounting devices 23 are hangers fixed to a ceiling wall 25 of the interior space 20. However, they may also be fixed to side walls or the bottom of the interior space 20. In Fig. 2, five of the six shown mounting devices 23 hold a surgical instrument 1, while the last mounting device 23 is empty. For better visibility, only one of the surgical instruments 1 and two of the mounting devices 23 are assigned reference numbers.

The mounting devices 23 are formed to accommodate the surgical instrument 1 tightly and hold them at a specified holding position and in a specified orientation. The specified orientation is slightly inclined, so that the longitudinal direction 30 of the surgical instrument 1 encloses an angle 32 with a vertical direction 31 of the cabinet 2. This angle 32 may be about 15°.

For each mounting device 23, the cabinet 2 comprises a nozzle 24 arranged in the ceiling wall 25 of the interior space 20. From these nozzles 24, a drying gas stream 26 is emitted at the control section 12 of the surgical instruments 1 in the mounting devices 23. Through this drying gas stream 26, the moisture in the gaps in between the control knobs 13 is removed efficiently, so that the drying time is shortened. In order to further decrease the drying time, the specified holding position is chosen to be close to the nozzle 24, for example about 5 cm. In addition, the inclined orientation of the control section 12 in the mounting device 23 further increases the drying efficiency, because the drying gas stream 26 is guided along and in between the control knobs 13 of the control section 12. The inclination of the mounting devices 23 and/or the distance of the mounting devices 23 to the nozzles 24 may be adjustable in order to allow an optimal drying for different surgical instruments 1.

A method for drying the surgical instruments 1 in the cabinet 2 may comprise different drying phases. For example, during a first phase, the drying gas stream 26 is emitted as a pulsed gas stream from the nozzle 24. For this pulsed gas stream, the peak pressure may be about 2 bar above ambient air pressure. This phase may comprise a single drying gas pulse or multiple pulses. Afterwards, the surgical instruments 1 may be further dried with a continuous gas stream with a constant gas pressure from the nozzle 24. The pressure is slightly reduced in this phase compared to the peak pressure during the gas pulses, but still lies well above the ambient air pressure. Afterwards, after the drying is complete, the surgical instruments 1 may be stored in the cabinet 2 during a storage phase, which may last up to 30 days. During this time, the pressure from the nozzle 24 is further reduced to a storage pressure, which is lower than the drying pressure during the continuous drying gas stream, but still above ambient air pressure to avoid recontamination.

The drying is controlled by a control system 29, which is represented as a control interface in Fig. 2. However, the control system 29 usually comprises a computer or a similar control unit connected to the different elements of the cabinet 2.

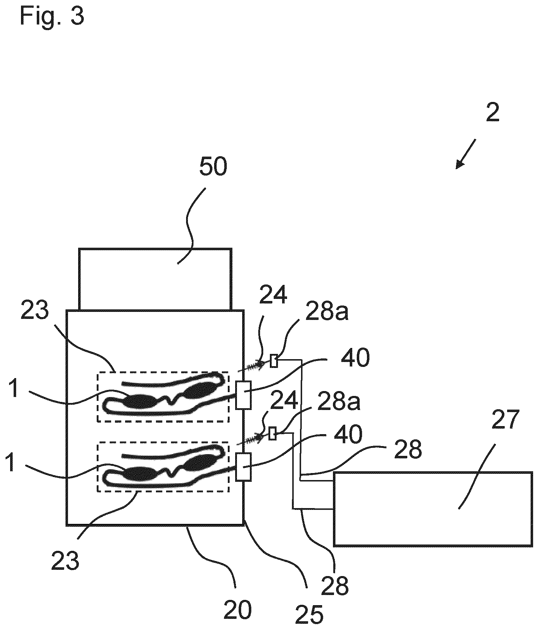

Fig. 3 shows a schematic representation of another embodiment of a cabinet 2 for drying the surgical instruments 1 after cleaning and disinfecting. In this case, the cabinet 2 is part of an apparatus for cleaning and disinfecting the surgical instruments 1. This means that the drying is done in the same apparatus that cleans and disinfects the surgical instruments 1. The interior space 20 of the cabinet 2 comprises two mounting devices 23, which may take the form of baskets. The surgical instruments 1 are connected to cleaning liquid supplies 40, which are used to clean the inner channels and/or the outer surface of the surgical instruments 1. Afterwards, the outer surface of the surgical instruments 1 needs to be dried off. In order to achieve this, the ceiling wall 25 or another wall comprises the nozzles 24, which again are aiming directly at the control section 12 of the surgical instruments 1 in the mounting device 23. The nozzles 24 are connected to a gas source 27 via supply lines 28. The gas source 27 may be configured to supply a pressurized gas stream. The gas may be ambient air, medical grade air or a gas composition, for example comprising noble gases. The gas may also comprise a temperature control unit not shown in Fig. 3, which is configured to heat and/or cool the gas in order to accelerate the drying. In order to control the timing of the drying gas stream 26, the cabinet 2 comprises two control elements 28a, for example valves. With these control elements 28a, the drying gas stream 26 may be turned on or off, for example to achieve the before mentioned gas pulses. The cabinet 2 may also comprise an air removal unit 50 connected to the interior space 20. The air removal unit 50 is utilized to regulate the pressure inside the interior space 20 and to remove access air from the interior space during and after drying. The gas source 27 may comprise a ventilator or a compressor. Pressurized air is air generated by a compressor, while the ventilator will supply regular, unpressurized air.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 1: surgical instrument
- 2: cabinet
- 4: endoscope
- 10: handle
- 11: tube
- 12: control section
- 13: control knobs
- 14: lever
- 15: button
- 16: control cog
- 17: control wheel
- 20: interior space
- 21: housing
- 22: door
- 23: mounting device
- 24: nozzle
- 25: ceiling wall
- 26: drying gas stream
- 27: gas source
- 28: supply line
- 28a: control element
- 29: control system
- 30: longitudinal direction
- 31: vertical direction
- 32: angle
- 40: cleaning liquid supply
- 50: air removal unit

## Claims

1. Cabinet (2) for drying surgical instruments (1), in particular endoscopes (4), wherein the cabinet (2) comprises an interior space (20) with at least one mounting device (23), wherein the mounting device (23) is configured to hold a surgical instrument (1) in a way, that a control section (12) of the surgical instrument (1) is arranged at a specified holding position and in a specified orientation, wherein the interior space (20) comprises at least one nozzle (24), wherein the nozzle (24) is connected to a gas source (27) by a supply line (28), wherein the gas source (27) is designed to supply a drying gas stream (26) to the nozzle (24) via the supply line (28), wherein the nozzle (24) is designed and arranged to guide the drying gas stream (26) directly towards the specified holding position, in such a direction that the drying gas stream (26) runs along and in between control knobs (13) of the control section (12), when the control section (12) is arranged at the specified holding position and in the specified orientation.

2. Cabinet (2) according to claim 1 comprising a control element (28a) arranged along the supply line (28), wherein the control element (28a) is designed to close and open the supply line (28), wherein in particular the control element (28a) is a valve.

3. Cabinet (2) according to claim 2 comprising a control system (29), wherein the control system (29) is configured to close and open the control element (28a) periodically in order to create a pulsed gas stream from the nozzle (24).

4. Cabinet (2) according to one of the claims 1 to 3, wherein the gas source (27) is an air source designed to supply heated air or unheated air to the nozzle (24).

5. Cabinet (2) according to one of the claims 1 to 4, wherein the gas source (27) supplies ambient air and/or medical grade air and/or a gas composition to the nozzle (24).

6. Cabinet (2) according to one of the claims 1 to 5, wherein the drying gas stream (26) is a pressurized gas stream.

7. Cabinet (2) according to one of the claims 1 to 6, wherein the mounting device (23) is configured to hold the control section (12) at an inclined orientation, such that a longitudinal direction (30) of the surgical instrument (1) at the specified holding position has an angle (32) of 0° to 20° to a vertical direction (31) of the cabinet (2) for a vertical drying cabinet and/or 20° to 45° to the vertical direction (31) of the cabinet (2) for a horizontal drying cabinet.

8. Cabinet (2) according to one of the claims 1 to 7, wherein the at least one nozzle (24) is arranged at a predetermined distance to the specified holding position, wherein the predetermined distance is smaller than 10 cm, wherein in particular the predetermined distance is 4 cm to 8 cm.

9. Cabinet (2) according to one of the claims 1 to 8, wherein the at least one nozzle (24) is arranged on a ceiling wall (25) of the interior space (20).

10. Cabinet (2) according to one of the claims 1 to 9 comprising a temperature control unit configured to control the temperature of the drying gas stream (26), wherein in particular the temperature control unit is configured to regulate the temperature of the drying gas stream (26) to the dew point of the drying gas stream (26).

11. Method for drying surgical instruments (1), in particular endoscopes (4), inside a cabinet (2), wherein the cabinet (2) comprises an interior space (20) with at least one mounting device (23), wherein the surgical instrument (1) is arranged in the at least one mounting device (23) in a way, that a control section (12) of the surgical instrument (1) is arranged at a specified holding position and in a specified orientation, wherein a gas source (27) supplies a drying gas stream (26) via a supply line (28) to a nozzle (24) of the interior space (20), wherein the nozzle (24) guides the drying gas stream (26) directly towards the specified holding position, in such a direction that the drying gas stream (26) runs along and in between control knobs (13) of the control section (12).

12. Method according to claim 11, wherein the supply line (28) is closed and opened via a control element (28a), in particular a valve, to stop and start the drying gas stream, wherein in particular the control element (28a) is closed and opened periodically to create a pulsed gas stream from the nozzle (24).

13. Method according to claim 11 or 12, wherein the gas source (27) is an air source, which supplies heated air or unheated air to the nozzle (24) and/or wherein the gas source (27) supplies ambient air and/or medical grade air and/or a gas composition to the nozzle (24) and/or wherein the drying gas stream (26) is a pressurized gas stream.

14. Method according to one of the claims 11 to 13, wherein the mounting device (23) holds the control section (12) at an inclined orientation, such that a longitudinal direction (30) of the surgical instrument (1) at the specified holding position has an angle (32) of 0° to 20° to a vertical direction (31) of the cabinet (2) for a vertical drying cabinet and/or 20° to 45° to the vertical direction (31) of the cabinet (2) for a horizontal drying cabinet and/or wherein the at least one nozzle (24) is arranged at a predetermined distance to the specified holding position, wherein the predetermined distance is smaller than 10 cm, wherein in particular the predetermined distance is 4 cm to 8 cm.

15. Method according to one of the claims 1 to 14, wherein the at least one nozzle (24) is arranged on a ceiling wall (25) of the interior space (20) and/or wherein a temperature control unit controls the temperature of the drying gas stream (26), wherein in particular the temperature control unit regulate the temperature of the drying gas stream (26) to the dew point of the drying gas stream (26).
